# EUROPÄISCHE PATENTSCHRIFT

(11) **EP 1 985 227 B1**
(45) Veröffentlichungstag und Bekanntmachung des Hinweises auf die Patenterteilung: **21.11.2012**
(21) Anmeldenummer: 07405129.3
(22) Anmeldetag: 27.04.2007
(51) Int. Cl.: A61B 3/13, A61B 19/00, G02B 15/04, G02B 21/22

(54) **Optikkomponente für ein Stereomikroskop**
Optical component for a stereo microscope
Composant optique pour un microscope stéreoscopique

(43) Veröffentlichungstag der Anmeldung: 29.10.2008
(73) Patentinhaber: HAAG-STREIT AG, CH-3098 Köniz (CH)
(72) Erfinder: Widmer, Hansruedi, 3145 Niederscherli (CH)
(74) Vertreter: Rüfenacht, Philipp Michael

(56) Entgegenhaltungen:
- DE-A1- 10 336 890
- DE-C2- 3 546 915
- GB-A- 2 255 651
- JP-A- 2001 169 168
- US-A- 5 371 557
- US-A- 5 956 536

## Beschreibung

### Technisches Gebiet

Die Erfindung betrifft eine Optikkomponente mit einem Paar von Strahlenwegen und einer Wechsleroptik, welche zwei Paare von Strahlenwegen umfasst, wobei die Wechsleroptik derart beweglich in der Optikkomponente befestigt ist, dass wahlweise die beiden Strahlenwege eines der Paare von Strahlenwegen der Wechsleroptik in einen Strahlabschnitt der beiden Strahlenwege der Optikkomponente einfügbar sind,

### Stand der Technik

Für die optische Untersuchung eines Auges eines Patienten stehen viele verschiedene Geräte zur Verfügung. Einige davon umfassen ein Mikroskop für die stereoskopische Betrachtung des Auges, seien dies vordere, hintere und/oder seitliche Augenabschnitte. Immer häufiger besteht jedoch die Anforderung, dass die vom Untersucher beobachteten Bilder des Auges gleichzeitig von einer weiteren Person betrachtet, auf einem Bildschirm in Echtzeit dargestellt oder auch elektronisch aufgezeichnet werden sollen. Zu diesem Zweck wird ein so genannter Strahlteiler in wenigstens einen der beiden Strahlengänge des Mikroskops eingefügt, welcher einen Teil oder die gesamte Strahlung aus dem entsprechenden Strahlengang zum Betrachter auskoppelt bzw. umlenkt und beispielsweise auf einen bildgebenden Sensor führt.

In der WO 2006/000072 (Mitre) ist ein derartiges Untersuchungsgerät beschrieben. In beide Strahlengänge des Stereomikroskops ist je ein halbtransparenter Spiegel eingefügt, welcher jeweils einen Teil der Strahlen auskoppelt und zu einer Kamera führt. Die von den Kameras aufgenommenen Bilder werden schliesslich auf einem Bildschirm dargestellt. Bei dieser Vorrichtung besteht das Problem, dass die Lichtmenge, die für die stereoskopische Betrachtung übrig bleibt, deutlich reduziert ist, da ein Teil der Strahlung für die Bildschirmdarstellung ausgekoppelt wird.

Die Auskopplung der Strahlen für die Bildschirmdarstellung kann beispielsweise auch so erfolgen, dass einer der beiden Strahlengänge vollständig zu einer Kamera umgelenkt wird, aber in diesem Fall verschwindet der stereografische Eindruck vollständig - sowohl bei der Betrachtung durch die Okulare als auch auf der Bildschirmdarstellung.

In der US 2002/0075449 A1 (Strahle) ist ein weiteres Stereomikroskop beschrieben. Dieses umfasst zwei Strahlbündel zur stereoskopischen Betrachtung durch einen ersten Beobachter sowie zwei zusätzliche Strahlbündel zur stereoskopischen Betrachtung durch einen zweiten Beobachter. Beide Beobachter haben die Möglichkeit, die Abbildungsvergrösserung mittels entsprechender, separater Vergrösserungssysteme einzustellen. Zwar steht hier für beide Betrachter jeweils die gesamte Lichtmenge der jeweiligen Strahlengänge zur Verfügung, aber das Mikroskop ist aufwändig und kompliziert aufgebaut, da es zwei Vergrösserungsvorrichtungen aufweist.

### Darstellung der Erfindung

Aufgabe der Erfindung ist es, eine dem eingangs genannten technischen Gebiet zugehörende Optikkomponente zu schaffen, welche einen einfachen Aufbau und eine einfache Handhabung eines damit ausgerüsteten optischen Untersuchungsgerätes ermöglicht.

Die Lösung der Aufgabe ist durch die Merkmale des Anspruchs 1 definiert. Die Optikkomponente umfasst ein Paar von Strahlenwegen, welche insbesondere derart angeordnet sind, dass sie eine stereoskopische Abbildung eines abzubildenden Objekts erlauben. Weiter umfasst die Optikkomponente eine Wechsleroptik, welche zwei Paare von Strahlenwegen umfasst. Die Wechsleroptik ist derart beweglich in der Optikkomponente befestigt, dass wahlweise eines der beiden Paare von Strahlenwegen dieser Wechsleroptik in einen Strahlabschnitt der beiden Strahlenwege der Optikkomponente einfügbar sind. D. h. die Wechsleroptik ermöglicht es, wahlweise verschiedene Abbildungen mit unterschiedlichen Abbildungseigenschaften des abzubildenden Objekts zu realisieren, indem jeweils eines der beiden Paare von Strahlenwegen der Wechsleroptik in den Strahlenweg der Optikkomponente eingefügt wird. Mit einer solchen Wechsleroptik können beispielsweise verschiedene Vergrösserungen der Abbildung erreicht werden. Es können aber auch andere Abbildungseigenschaften wie beispielsweise die Grösse des ausgeleuchteten Bereichs des Objekts, die Beleuchtungsstärke, das Beleuchtungsspektrum, die Grösse des erzeugten Bildes, die Helligkeit der Abbildung, die Absorption bestimmter Wellenlängen oder beliebige andere Eigenschaften variiert werden.

Gemäss der Erfindung umfasst die Optikkomponente nun wenigstens einen, den beiden Strahlenwegen für die stereoskopische Abbildung zugeordneten, zusätzlichen Strahlenweg und die Wechsleroptik umfasst pro Paar von Strahlenwegen ebenfalls wenigstens einen, dem jeweiligen Paar zugeordneten und räumlich getrennt von diesem angeordneten, zusätzlichen Strahlenweg. Weiter ist die Wechsleroptik derart ausgebildet, dass beim wahlweisen Einfügen der beiden Strahlenwege eines der beiden Paare von Strahlenwegen der Wechsleroptik in die beiden Strahlenwege der Optikkomponente, gleichzeitig der diesem Paar zugeordnete, zusätzliche Strahlenweg der Wechsleroptik in einen Strahlabschnitt des zusätzlichen Strahlenweges der Optikkomponente eingefügt wird. D. h. in einer ersten Stellung der Wechsleroptik ist das erste Paar von Strahlenwegen mit dem zugeordneten zusätzlichen Strahlenweg aktiv und in einer zweiten Stellung der Wechsleroptik ist das zweite Paar von Strahlenwegen mit dem zugeordneten zusätzlichen Strahlenweg aktiv.

Der Begriff "zugeordnet" bedeutet in diesem Zusammenhang unter anderem, dass sich mit dem zusätzlichen Strahlenweg gleichzeitig derselbe Bereich des abzubildenden Objekts beobachten bzw. beleuchten lässt, der auch mit den Strahlenwegen für die erste Abbildung abgebildet bzw. beleuchtet wird.

Eine solche Optikkomponente dient zum Variieren der Eigenschaften der optischen Abbildung (nachfolgend auch als erste Abbildung bezeichnet). Sie kann in verschiedenen Geräten wie beispielsweise Mikroskopen oder ähnlichen Geräten verwendet werden, mit welchen die stereoskopische Abbildung eines Gegenstands wie z. B. eines Auges eines Patienten möglich ist. Die beiden Strahlenwege der Optikkomponente werden hierbei typischerweise auf die beiden Okulare eines solchen Mikroskops geführt. Der zusätzliche Strahlenweg dient beispielsweise dazu, eine zusätzliche Abbildung des abzubildenden Objekts oder Gegenstands zu erzeugen, etwa für die Betrachtung durch einen zweiten Betrachter oder für eine Darstellung auf einem Bildschirm oder zur (elektronischen) Speicherung der so erzeugten Bilder. Der zusätzliche Strahlenweg kann aber auch zur Beleuchtung des abzubildenden Objekts verwendet werden.

Indem sowohl die Strahlenwege für die erste Abbildung als auch der zusätzliche Strahlenweg in einer einzigen Optikkomponente vereint werden, kann diese kompakt und entsprechend platzsparend und kostengünstig ausgebildet werden. Dadurch kann die Komplexität der Optikkomponente als solche, als auch diejenige der optischen Geräte, in welche solche Optikkomponenten eingesetzt werden, reduziert und zudem die Bedienung dieser Geräte vereinfacht werden, da sich die Eigenschaften der ersten und der zusätzlichen Abbildung gleichzeitig und aufeinander abgestimmt verändern lassen, indem die Wechsleroptik von der ersten in die zweite Stellung (oder umgekehrt) gebracht wird.

Mit einem zusätzlichen Strahlenweg der Optikkomponente (und entsprechend einem zusätzlichen Strahlenweg pro Paar von Strahlenwegen der Wechsleroptik) lässt sich beispielsweise eine zusätzliche monoskopische Bildschirmdarstellung des abzubildenden Objekts erreichen. Je nach Anwendung ist es jedoch von Vorteil, wenn auch diese zusätzliche Abbildung stereoskopisch erfolgen kann. Für diesen Fall umfasst die Optikkomponente nicht nur einen zusätzlichen, sondern vorzugsweise genau zwei zusätzliche Strahlenwege. Die Wechsleroptik umfasst entsprechend auch zwei zusätzliche Strahlenwege pro Paar von Strahlenwegen. Bei einer solchen Ausführungsform der Erfindung ist aber mit diesen beiden zusätzlichen Strahlenwegen nicht nur eine zusätzliche, stereoskopische Abbildung möglich, es ist auch möglich, diese beiden zusätzlichen Strahlenwege für verschiedene Zwecke zu benutzen. So kann beispielsweise einer für eine zusätzliche monoskopische Abbildung und der zweite für eine weitere, zusätzliche monoskopische Abbildung verwendet werden. Einer oder auch beide dieser zusätzlichen Strahlenwege könnten aber auch für eine Beleuchtung des abzubildenden Objekts verwendet werden.

Selbstverständlich ist es auch möglich, drei oder mehr zusätzliche Strahlenwege vorzusehen, um weitere monoskopische oder stereoskopische Abbildungen zu erzeugen oder das abzubildende Objekt (beispielsweise mit unterschiedlichen Beleuchtungsquellen) zu beleuchten. Damit der Platzbedarf jedoch nicht allzu hoch und die Optikkomponente nicht allzu gross wird, weist diese vorzugsweise lediglich zwei zusätzliche Strahlenwege auf.

Bei einer solchen Optikkomponente mit vier Strahlenwegen (zwei Strahlenwegen für die erste Abbildung und zwei zusätzlichen Strahlenwegen) weist die Wechsleroptik pro Stellung ebenfalls vier Strahlenwege auf. Diese vier Strahlenwege pro Stellung der Wechsleroptik können im Prinzip beliebig angeordnet werden. Eine einfache Art, deren gegenseitige Lage zu charakterisieren, ist die gegenseitige Lage der beiden Ebenen, welche durch jeweils zwei zusammengehörige Strahlenwege eines Paares bzw. deren Strahlachsen definiert werden. (An dieser Stelle wird davon ausgegangen, dass die beiden zusammengehörigen Strahlenwege eines Paares jeweils tatsächlich eine Ebene aufspannen. Der Fall, in welchem dies nicht geschieht, wird hier nicht berücksichtigt, da dies die optische Abbildung verunmöglichen oder zumindest stark erschweren würde.) Die beiden Strahlenwege jedes Paares von Strahlenwegen der Wechsleroptik definieren beispielsweise eine erste und die beiden diesem Paar zugeordneten zusätzlichen Strahlenwege definieren eine zweite Ebene. Diese beiden Ebenen können beispielsweise parallel zueinander liegen, wobei sie entweder in einem bestimmten Abstand zueinander liegen oder identisch sind, d. h. eine gemeinsame Ebene definieren. Die beiden Ebenen können aber auch einen Winkel einschliessen, sodass sie sich in genau einer Geraden schneiden. Vorzugsweise sind die Strahlenwege derart angeordnet, dass sich die beiden Ebenen in einer Geraden (nachfolgend als Schnittgerade bezeichnet) schneiden, welche parallel zur optischen Achse der Optikkomponente liegt.

Die Strahlenwege können beispielsweise nun derart angeordnet sein, dass diese Schnittgerade ausserhalb der beiden ersten Strahlenwege liegt und die beiden Ebenen einen Winkel verschieden von 90 Grad einschliessen. In bevorzugter Weise sind die Strahlenwege aber derart angeordnet, dass die beiden Ebenen senkrecht zueinander stehen und die Schnittgerade der beiden Ebenen zwischen den Strahlenwegen für die erste Abbildung liegt. Diese Anordnung der Strahlenwege erlaubt eine möglichst Platz sparende Konstruktion der Optikkomponente, wobei je nach Anwendung beispielsweise auch der Abstand der beiden jeweils zusammengehörigen Strahlenwege optimiert werden kann.

Wie erwähnt handelt es sich bei der Optikkomponente um eine Vorrichtung, mit welcher gleichzeitig die Abbildungseigenschaften der ersten sowie der zusätzlichen Abbildung verändert werden können. Beispielsweise kann die Wechsleroptik derart ausgebildet sein, dass die erste Abbildung sowie die zusätzliche Abbildung in einer ersten Stellung der Wechsleroptik mit einer ersten Vergrösserung erfolgt und in einer zweiten Stellung der Wechsleroptik eine andere Vergrösserung der ersten Abbildung sowie der zusätzlichen Abbildung erreicht werden kann. Eine solche Optikkomponente wird daher nachfolgend auch als Wechselvorrichtung bezeichnet. Hierbei ist zu beachten, dass unter dem Begriff Vergrösserung im Rahmen der vorliegenden Erfindung auch eine Vergrösserung mit dem Faktor eins verstanden werden soll.

Zum Wechseln der Abbildungseigenschaften zwischen der jeweils ersten und der jeweils zugehörigen zusätzlichen Abbildung umfasst die Wechsleroptik für jeden Strahlenweg vorzugsweise wenigstens eine Abbildungsoptik, welche wahlweise in die betreffenden Strahlenwege eingefügt bzw. daraus entfernt werden kann. Bei einer solchen Ausführungsform muss man sich zwar im Voraus auf einige wenige fest eingestellte Abbildungseigenschaften wie z. B. Vergrösserungsfaktoren festlegen, die entsprechenden Abbildungsoptiken können allerdings mit hoher Qualität, beispielsweise mit einer hohen Lichtstärke, hergestellt werden. Um verschiedene Vergrösserungen zu erreichen, wäre zwar auch der Einsatz von stufenlos variablen Abbildungsoptiken wie beispielsweise Zoom-Systemen möglich. Im Gegensatz zu den Vergrösserungsoptiken mit fixer Brennweite/Vergrösserung haben diese meist jedoch eine geringere Lichtstärke und der genaue Vergrösserungsfaktor lässt sich auch nicht immer mit genügender Genauigkeit bestimmen.

Derartige Abbildungsoptiken umfassen beispielsweise je eine Linsenanordnung mit einer oder mehrere Linsen.

Die verschiedenen Abbildungsoptiken können beispielsweise derart ausgebildet sein, dass sie einzeln in einen Strahlenweg eingefügt, z. B. radial zum Strahlenweg in diesen eingeschoben werden können. Zum Ändern der Vergrösserung müsste dann eine eingesetzte Abbildungsoptik entfernt und eine andere eingesetzt werden. In einem anderen Ausführungsbeispiel könnten mehrere Abbildungsoptiken beispielsweise nebeneinander auf einer Art Schiene befestigt werden, wobei die Schiene so positioniert beispielsweise von der Seite her eingeschoben wird, bis sich die der gewünschten Vergrösserung entsprechende Abbildungsoptik in dem betreffenden Strahlenweg befindet. Da die Vergrösserung jedoch nicht nur für einen, sondern mindestens drei Strahlenwege einstellbar sein muss, wären solche Konstruktionen für deren Benutzer ziemlich unhandlich.

Bei einer bevorzugten Ausführungsform der Erfindung umfasst die Wechsleroptik daher eine Trägervorrichtung, welche um eine Drehachse drehbar ist. Sämtliche Abbildungsoptiken sind hierbei an dieser Trägervorrichtung befestigt, wobei sie derart befestigt sind, dass durch das Drehen der Trägervorrichtung jeweils eine der Abbildungsoptiken in die Strahlenwege eingefügt wird. D. h. je nach Stellung, in diesem Fall je nach Winkelposition der Trägervorrichtung, befindet sich eine der für einen bestimmten Strahlenweg vorgesehenen Abbildungsoptiken in dem jeweiligen Strahlenweg.

Zur Auswahl der gewünschten Stellung der Wechsleroptik, d. h. zum Drehen der Trägervorrichtung, umfasst die Wechsleroptik vorzugsweise Einstellmittel wie z. B. einen Drehknopf oder Drehschalter, welcher insbesondere von Hand betätigbar, d. h. drehbar, ist. Die Trägervorrichtung ist mit Vorteil derart ausgebildet, dass durch eine Drehung der Trägervorrichtung mit Hilfe der Einstellmittel die Abbildungseigenschaften in sämtlichen einander zugeordneten Strahlenwegen gleichzeitig eingestellt bzw. verändert wird. Es ist jedoch nicht zwingend, dass bei einer bestimmten Stellung der Trägervorrichtung für jeden Strahlenweg dieselben Abbildungseigenschaften resultieren. Die Abbildungsoptiken können beispielsweise so ausgebildet sein, dass für die beiden Strahlenwege für die erste Abbildung ein erster Vergrösserungsfaktor und für die beiden zusätzlichen Strahlenwege ein zweiter Vergrösserungsfaktor resultiert, welcher gleich oder verschieden zum ersten Vergrösserungsfaktor ist.

Bei einem anderen Ausführungsbeispiel ist die Trägervorrichtung so ausgebildet, dass die Abbildungsoptiken die ersten Strahlenwege für die erste Abbildung und die Abbildungsoptiken für die beiden zusätzlichen Strahlenwege unabhängig voneinander um die Drehachse gedreht werden können. In diesem Fall müssten auch die Einstellmittel so ausgebildet sein, dass damit wahlweise die Abbildungsoptiken für die beiden ersten Strahlenwege, die Abbildungsoptiken für die beiden zusätzlichen Strahlenwege oder auch sämtliche Abbildungsoptiken gleichzeitig um die Drehachse gedreht werden können.

Auch eine automatische/maschinelle Betätigung der Einstellmittel ist grundsätzlich denkbar, wobei die gewünschte Vergrösserung beispielsweise mit Hilfe eines Computers ausgewählt werden kann und der Computer eine entsprechende Betätigungsvorrichtung wie etwa einen mit den Einstellmitteln verbundenen Motor derart ansteuert, dass die Trägervorrichtung in die entsprechende Position gedreht wird.

Die gesamte Optikkomponente ist vorzugsweise in ein Gehäuse integriert. Dieses umfasst mehrere Öffnungen, durch welche die Strahlen vom bzw. zum Objekt in das Gehäuse ein-, respektive daraus austreten können. Grundsätzlich wäre es natürlich möglich, für jeden Strahlenweg eine eigene Ein- bzw. Austrittsöffnung vorzusehen. Um jedoch die Herstellung zu vereinfachen, umfasst das Gehäuse eine objektseitig angeordnete Öffnung für die Strahlenwege der ersten und der zusätzlichen Abbildung. Weiter umfasst das Gehäuse wenigstens eine erste, beobachterseitig angeordnete Öffnung, für die Strahlenwege der ersten Abbildung sowie wenigstens eine zweite, beobachterseitig angeordnete Öffnung für die Strahlenwege der zusätzlichen Abbildung. Die beiden beobachterseitig angeordneten Öffnungen sind hierbei vorzugsweise räumlich getrennt voneinander angeordnet.

Um zu verhindern, dass Staubpartikel oder ähnliches die optischen Systeme innerhalb des Gehäuses verschmutzen, sind diese Öffnungen typischerweise mit einem optisch transparenten Material, beispielsweise mit entsprechend geformten Glasscheiben, verschlossen, wobei diese gleichzeitig auch als Teil der optischen Systeme, beispielweise als Objektivlinse des zugehörigen Mikroskops, dienen können.

Wie soeben beschrieben, sind die beiden beobachterseitigen Öffnungen typischerweise voneinander räumlich getrennt angeordnet. Der Hauptgrund dafür ist, dass die entsprechenden Strahlenwege in der Regel getrennt voneinander weiterverarbeitet werden. Diese Trennung kann beispielsweise erreicht werden, indem der Abstand zwischen den ersten beiden Strahlenwegen und den zusätzlichen Strahlenwegen einfach genügend gross gewählt wird. Dadurch vergrössern sich aber auch die geometrischen Abmessungen des Gehäuses der Optikkomponente, weshalb diese nicht in der geforderten Kompaktheit ausgebildet werden kann.

Vorzugsweise umfasst die Optikkomponente daher eine Umlenkvorrichtung, mit welcher die Strahlenwege der zusätzlichen Abbildung zur zweiten, beobachterseitig angeordneten Öffnung umgelenkt werden. Auch diese Umlenkvorrichtung, welche beispielsweise einen oder mehrere Spiegel umfasst, ist typischerweise in dem Gehäuse der Optikkomponente angeordnet, obwohl diese Umlenkung auch ausserhalb des Gehäuses erfolgen könnte. D. h. die Strahlenwege der zusätzlichen Abbildung verlaufen innerhalb des Gehäuses im Wesentlichen in der Nähe der Strahlenwege der ersten Abbildung und werden dann von der Umlenkvorrichtung ab- bzw. umgelenkt, sodass sie nicht zusammen mit den Strahlenwegen der ersten Abbildung aus der ersten beobachterseitig angeordneten Öffnung, sondern aus der zweiten beobachterseitig angeordneten Öffnung austreten.

Selbstverständlich könnte die Umlenkvorrichtung auch derart ausgebildet und angeordnet sein, dass nicht die Strahlenwege der zusätzlichen Abbildung, sondern jene der ersten Abbildung umgelenkt werden.

Eine Optikkomponente wie vorgängig beschrieben, kann wie bereits erwähnt in verschiedenen Geräten eingesetzt werden. Sie kann beispielsweise in einem Greenough Stereomikroskop verwendet werden. Bei einem solchen Mikroskop stehen die beiden Strahlenwege für das linke und das rechte Auge des Betrachters typischerweise einen Winkel in der Grössenordnung von 15° ein. Es existiert in der Regel kein Bereich, in welchem die beiden Strahlenwege parallel verlaufen. Bei einer Wechselvorrichtung für ein solches Greenough-Mikroskop sind die Abbildungsoptiken derart angeordnet, dass sowohl die beiden Strahlenwege für die erste Abbildung als auch jene für die zusätzliche Abbildung einen entsprechenden Winkel einschliessen. Auch sind die optischen Komponenten derart dimensioniert, dass im Auge des Betrachters (oder beispielsweise auf einem entsprechend positionierten CCD-Chip) das gewünschte Bild entsteht.

Vorzugsweise wird eine solche Optikkomponente jedoch in einem Gerät eingesetzt, bei welchem die beiden Strahlenwege für die erste Abbildung in zumindest einem Abschnitt parallel verlaufen. Dies ist insbesondere bei Mikroskopen nach dem Fernrohr-Prinzip der Fall. Bei solchen Geräten wird die Wechselvorrichtung mit Vorteil in jenem Bereich des Geräts eingesetzt, in welchem die Strahlen parallel verlaufen. Entsprechend verlaufen auch die Strahlenwege eines Paares von Strahlenwegen der Wechsleroptik parallel zueinander sowie parallel zu den diesem Paar zugeordneten zusätzlichen Strahlenwege.

Bei einem bevorzugten Ausführungsbeispiel einer solchen Wechselvorrichtung ist jede Abbildungsoptik für eine optische Abbildung von unendlich nach unendlich ausgebildet, wobei die Abbildung vorzugsweise aufrecht ist. Mit einem Galilei-System lässt sich beispielsweise eine solche Abbildungsoptik realisieren. Ein Galilei-System umfasst eine Linsenanordnung mit einer Sammellinse auf der einen und einer Zerstreuungslinse auf der anderen Seite, wobei die einfallenden Strahlen parallel verlaufen und die ausgehenden Strahlen nach der Vergrösserung (bzw. Verkleinerung) wiederum parallel verlaufen. Die Trägervorrichtung umfasst daher eine Mehrzahl solcher Galilei-Systeme mit jeweils mindestens zwei Linsen pro Strahlenweg und wird deshalb auch als Linsentrommel bezeichnet. Bei einer Wechsleroptik mit vier Strahlenwegen pro Stellung umfasst eine solche Linsentrommel entsprechend auch vier Galilei-Systeme pro Stellung. Jeweils eines für jeden der vier Strahlenwege (zwei Strahlenwege für die erste und zwei für die zusätzliche Abbildung pro Stellung der Wechsleroptik).

Diese Abbildung von unendlich nach unendlich ermöglicht es, dass eine beliebige Abbildungsoptik oder gar keine Abbildungsoptik (Vergrösserungsfaktor eins) in einen Strahlenweg eingefügt werden kann. D. h. in jedem Strahlenweg kann ein Galilei-System (durch einfaches Drehen der Linsentrommel) durch ein anderes Galilei-System ersetzt werden, ohne dass sonstige weitere Anpassungen notwendig wären.

Allerdings könnten anstelle von Galilei-Systemen auch andere Abbildungsoptiken verwendet werden, welche eine Abbildung mit parallel einfallenden und ausgehenden Strahlen ermöglichen. Hierzu gehören beispielsweise die so genannten Abbildungssysteme nach Kepler. Bei solchen Systemen entsteht jedoch kein aufrechtes Bild, sodass der Benutzer entweder mit einem umgedrehten Bild arbeiten muss oder weitere Mittel vorgesehen sein müssen, um das Bild nochmals zu drehen.

Wie bereits erwähnt, wird eine solche Optikkomponente vorzugsweise mit einem optischen Untersuchungsgerät, wie etwa einem Stereomikroskop, verwendet. Ein solches Gerät umfasst typischerweise zwei Strahlenwege für eine stereoskopische Abbildung eines abzubildenden Objekts. Die Optikkomponente ist dann wahlweise derart mit dem Untersuchungsgerät verbind- bzw. daran anschliessbar dass die beiden Strahlenwege eines Paares von Strahlenwegen der Optikkomponente mit einem Abschnitt der beiden Strahlenwege des Untersuchungsgeräts zusammenfallen. Entsprechend fallen zwei weitere Strahlenwege des Untersuchungsgerätes von dem bzw. zu dem abzubildenden Objekt mit dem bzw. den zusätzlichen Strahlenwegen der Optikkomponente zusammen.

Ein optisches Untersuchungsgerät gemäss der Erfindung umfasst folglich zwei Strahlenwege für eine stereoskopische Betrachtung eines abzubildenden Objekts sowie eine Optikkomponente bzw. Wechselvorrichtung wie sie vorgängig beschrieben worden ist. Solche Untersuchungsgeräte werden z. B. häufig zur Augenuntersuchung eingesetzt. Wie bereits erwähnt, kann es sich beispielsweise um ein Stereomikroskop nach Greenough oder eines nach dem Fernrohr-Prinzip handeln. Letztere umfassen, wie oben erwähnt, zumindest einen Abschnitt mit parallel verlaufenden Strahlenwegen. Dieser Abschnitt wird nachfolgend auch als Parallelabschnitt bezeichnet.

Ein solches optisches Untersuchungsgerät mit einem Parallelabschnitt umfasst typischerweise eine Objektivlinse sowie zwei Okulare, d. h. für jeden der beiden (zumindest teilweise parallelen) Strahlenwege ein separates Okular, durch welche hindurch der Betrachter die stereoskopische Abbildung des abzubildenden Objekts betrachten kann. Bei einem solchen Gerät ist die Wechselvorrichtung in dem Parallelabschnitt nun derart zwischen der Objektivlinse und den Okularen in die Strahlenwege des optischen Untersuchungsgeräts einfügbar, dass die beiden parallelen Strahlenwege für die erste optische Abbildung der Wechselvorrichtung je einen Abschnitt der beiden Strahlenwege des optischen Untersuchungsgerätes bilden. D. h. ein Lichtstrahl, welcher in Richtung eines der beiden Strahlenwege des Untersuchungsgerätes durch die Objektivlinse in das Gerät eintritt, verläuft entlang diesem Strahlenweg, tritt durch die objektseitig angeordnete Öffnung der Wechselvorrichtung in deren Gehäuse ein, verlässt dieses wieder durch die erste beobachterseitig angeordnete Öffnung und folgt dann wiederum dem Strahlenweg des Geräts bis zu dem entsprechenden Okular.

Entsprechend umfasst das Untersuchungsgerät auch wenigstens einen weiteren Strahlenweg, welcher dem zusätzlichen Strahlenweg der Wechselvorrichtung entspricht. D. h. der wenigstens eine zusätzliche Strahlenweg der Wechselvorrichtung bildet quasi einen Abschnitt dieses wenigstens einen weiteren Strahlenwegs des Untersuchungsgeräts.

Bei der Verwendung dieser Strahlenwege zur Erzeugung einer beobachtbaren Abbildung verläuft der Energiefluss vom Objekt zum Beobachter. Wird einer dieser Strahlenwege allerdings für die Beleuchtung des abzubildenden Objekts verwendet, verläuft der Energiefluss in umgekehrter Richtung von einer Lichtquelle hin zum abzubildenden bzw. zu beleuchtenden Objekt. D. h. das von einer Lichtquelle ausgehende Licht tritt durch eine der beobachterseitig angeordneten Öffnungen in das Gehäuse der Wechselvorrichtung ein, durchquert die Wechsleroptik, verlässt das Gehäuse durch die objektseitig angeordnete Öffnung und wird zum zu beleuchtenden Objekt geführt, beispielsweise einem zu untersuchenden Auge.

Anstelle von Okularen können bei dem Untersuchungsgerät allerdings auch andere optische Systeme vorgesehen sein oder die Abbildungsstrahlen werden z. B. auf einen bildgebenden Sensor geführt. In diesem Fall erfolgt die Betrachtung des Objektbildes nicht durch Okulare, sondern beispielsweise an einem Bildschirm, auf welchem die von einem solchen bildgebenden Sensor erfassten Bilder dargestellt werden.

Wie bereits erwähnt, können der bzw. die zusätzlichen Strahlenwege beispielsweise für einen zusätzlichen Betrachter auf entsprechende, zusätzliche Okulare geführt werden. Bei einer bevorzugten Ausführungsform der Erfindung umfasst das optische Untersuchungsgerät jedoch eine optische Abbildungsvorrichtung, wobei der wenigstens eine zusätzliche Strahlenweg der Wechselvorrichtung zu dieser Abbildungsvorrichtung umlenkbar ist. Die Abbildungsvorrichtung umfasst beispielsweise ein Ophthalmoskop, z. B ein digitales Ophthalmoskop, bei welchem der abzubildende Bereich abgetastet wird. So lassen sich beispielsweise Live-Bilder vom abzubildenden Objekt erzeugen. In englisch wird ein solches Ophthalmoskop auch als "scanning digital ophthalmoscope" bezeichnet.

Wird ein solches Ophthalmoskop beispielsweise zusammen mit einem erfindungsgemässen Untersuchungsgerät für die Augenuntersuchung eingesetzt, ermöglicht dies einerseits eine Untersuchung des Auges durch einen Benutzer mittels der Okulare des Geräts, sowie gleichzeitig eine Darstellung von Live-Bildern des Auges, beispielsweise auf einem an die Abbildungsvorrichtung angeschlossenen Bildschirm, oder auch die Speicherung solcher Bilder auf einem geeigneten Speichermedium. Und dies bei voller Lichtausbeute für jede dieser Darstellungen, d. h. ohne eine verminderte Lichtausbeute durch eine Teilung bzw. Auskopplung der von dem Auge ausgehenden Lichtstrahlen.

Da das natürliche Licht häufig nicht ausreicht, um das abzubildende Objekt ausreichend zu beleuchten, umfassen solche Untersuchungsgeräte häufig entsprechende Beleuchtungsmittel. Bei einigen Geräten, wie z. B. bei einem Spaltlampenmikroskop, ist zwingend eine Beleuchtung vorzusehen. Diese Beleuchtung erfolgt bei solchen Geräten seitlich oder von oben bzw. unten. Bei anderen Geräten werden die Beleuchtungsstrahlen auch in die Beobachtungsstrahlengänge eingekoppelt, wobei hierfür entsprechende Koppelvorrichtungen, beispielsweise halbtransparente Spiegel, verwendet werden.

Bei einer weiteren bevorzugten Ausführungsform der Erfindung wird wenigstens einer der zusätzlichen Strahlenwege zur Beleuchtung des abzubildenden Objekts verwendet, wofür das Untersuchungsgerät bzw. die vorgängig erwähnte Abbildungsvorrichtung entsprechende Beleuchtungsmittel umfasst.

Indem für die Beleuchtung des Objekts einer oder mehrere der zusätzlichen Strahlenwege verwendet wird/werden, kann wiederum vermieden werden, dass wie beim Stand der Technik entsprechende Spiegel in den Beobachtungsstrahlenweg eingefügt werden müssen. D. h. auch in diesem Fall kann vermieden werden, dass für die zusätzliche Beleuchtung des Objekts die Lichtmenge für die stereoskopische Betrachtung des Objekts via die Beobachtungsstrahlenwege reduziert werden muss.

Bei einer bevorzugten Ausführungsform eines derartigen optischen Untersuchungsgerätes umfasst die Wechsleroptik pro Stellung zwei zusätzliche Strahlenwege, sodass beispielsweise einer davon für die Beleuchtung mit einer Beleuchtungsvorrichtung des Ophthalmoskops und der andere für die Erzeugung der Live-Bilder verwendet werden kann.

Die Beleuchtungsstrahlen verlaufen hierbei in der Wechselvorrichtung in der entgegengesetzten Richtung wie die Beobachtungsstrahlen, nämlich ausgehend von der Beleuchtungsquelle hin zum Objekt.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Eine perspektivische Darstellung einer Linsentrommel für einen erfindungsgemässen Vergrösserungswechsler mit drei Vergrösserungsstufen;
- Fig. 2: eine schematische Darstellung einer weiteren Linsentrommel für drei Vergrösserungsstufen;
- Fig.3: eine schematische Darstellung einer weiteren Linsentrommel für fünf Vergrösserungsstufen;
- Fig.4: eine schematische Darstellung eines Vergrösserungswechslers mit der Linsentrommel aus Fig. 3;
- Fig. 5: eine perspektivische Darstellung eines erfindungsgemässen Vergrösserungswechslers;
- Fig. 6: eine schematische Darstellung des Strahlengangs eines erfindungsgemässen Untersuchungsgerätes;
- Fig. 7: eine schematische Darstellung eines erfindungsgemässen Untersuchungsgerätes mit einem, an den Vergrösserungswechsler angeschlossenen, digitalen Ophthalmoskop;
- Fig. 8: eine weitere schematische Darstellung des Untersuchungsgeräts aus Fig. 7;
- Fig. 9: eine schematische Darstellung der Seitenansicht einer Linsentrommel für ein Stereomikroskop nach Greenough in einer ersten Stellung;
- Fig. 10: eine schematische Darstellung der Linsentrommel aus Fig. 9 in einer Ansicht von oben;
- Fig. 11: eine schematische Darstellung der Linsentrommel aus Fig. 9 in einer zweiten Stellung und
- Fig. 12: eine schematische Darstellung der Linsentrommel aus Fig. 11 in einer Ansicht von oben.

Grundsätzlich sind in den Figuren gleiche Teile mit gleichen Bezugszeichen versehen.

### Wege zur Ausführung der Erfindung

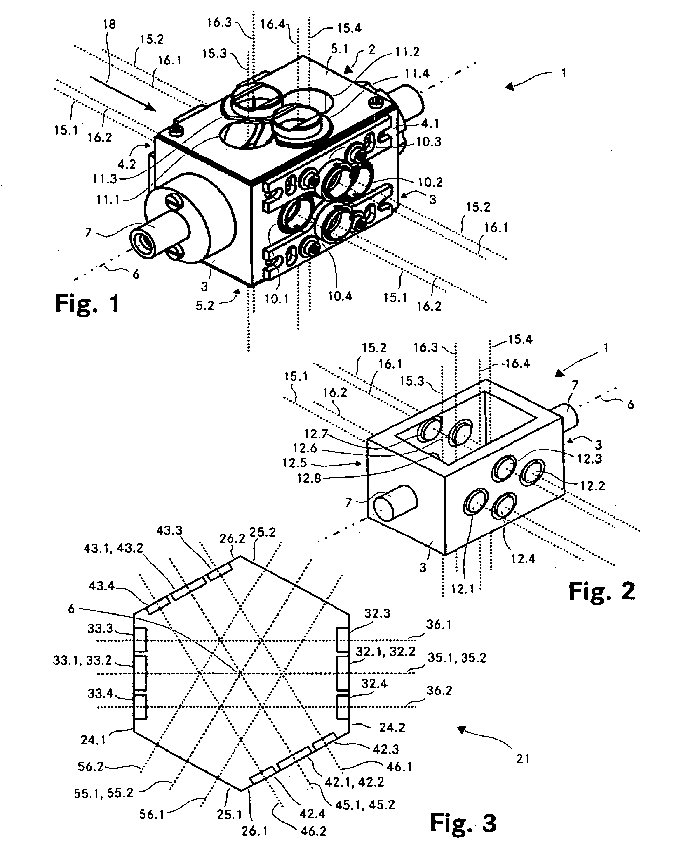

In Fig. 1 ist in einer perspektivischen Ansicht eine Linsentrommel 1 für eine erfindungsgemässe Wechsleroptik, in diesem Fall für einen Vergrösserungswechsler, dargestellt. Die Linsentrommel 1 umfasst ein quaderförmiges Gehäuse 2 mit zwei im Wesentlichen quadratischen Stirnwänden bzw. Stirnflächen 3, sowie zwei ersten Seitenwänden bzw. Seitenflächen 4.1, 4.2 und zwei zweiten Seitenwänden bzw. Seitenflächen 5.1, 5.2. Damit die Linsentrommel 1 um eine Drehachse 6 drehbar ist, ist an jeder Stirnfläche 3 ein entsprechendes Achsstück 7 angeflanscht, mit welchen die Linsentrommel 1 im Vergrösserungswechsler gelagert wird.

In der Seitenfläche 4.1 sind nun Öffnungen 10.1, 10.2, 10.3, 10.4 vorgesehen, wobei in der Seitenfläche 4.2 ebenfalls entsprechende Öffnungen (nur teilweise sichtbar) vorgesehen sind. Durch jeweils zwei sich entsprechende Öffnungen in den sich gegenüberliegenden Seitenflächen 4.1 und 4.2 verlaufen nun die Strahlenwege 15.1, 15.2 bzw. 16.1, 16.2.

Auch in den Seitenflächen 5.1, 5.2 sind derartige Öffnungen 11.1, 11.2, 11.3, 11.4 vorgesehen, welche entsprechende Strahlenwege 15.3, 15.4 bzw. 16.3, 16.4 definieren.

Die Öffnungen 10.1, 10.2, 10.3, 10.4 und damit auch die entsprechenden Strahlenwege 15.1, 15.2 und 16.1, 16.2 bzw. 15.3, 15.4 und 16.3, 16.4 sind hierbei in Kreuzform angeordnet. D. h. die Strahlenwege 15.1, 15.2 spannen eine erste Ebene und die Strahlenwege 16.1, 16.2 spannen eine zweite Ebene auf. Diese beiden Ebenen stehen senkrecht zueinander, wobei die Schnittgerade der beiden Ebenen parallel zu den Strahlenwegen 15.1, 15.2, 16.1, 16.2 liegt und zwar so, dass sie zwischen den Strahlenwegen 15.1, 15.2 und auch zwischen den Strahlenwegen 16.1, 16.2 liegt. Entsprechendes gilt für die Strahlenwege 15.3, 15.4, 16.3, 16.4 in den Seitenflächen 5.1 und 5.2.

in die Öffnungen 10.1, 10.2, 10.3, 10.4 wie auch in die Öffnungen auf der gegenüberliegenden Seitenfläche 4.2 ist nun jeweils eine Linse 12.1, 12.2, 12.3, 12.4 ein- bzw. von aussen oder innen aufgesetzt, sodass die zwei Linsen, die in sich gegenüberliegenden Öffnungen liegen, jeweils eine Vergrösserungsoptik, ein so genanntes Galilei-System, bilden (diese wurden weiter oben als Abbildungsoptik bezeichnet). Unter dem Begriff Linse wird jedoch nicht nur eine einzelne Linse verstanden, er kann jeweils auch eine Kombination von Linsen umfassen. Solche Linsen werden in den Öffnungen typischerweise mittels entsprechender Linsenfassungen befestigt.

Auf diese Weise liegt in jedem der Strahlenwege 15.1, 15.2, 16.1, 16.2 jeweils ein separates Galilei-System. Durch die Wahl der Linsen und deren Abstände können die Vergrösserungsfaktoren im Prinzip für jedes Galilei-System unterschiedlich gewählt werden. Typischerweise sind jedoch zumindest die Vergrösserungsfaktoren der beiden Galilei-Systeme in den beiden Strahlenwegen 15.1, 15.2 gleich, da diese meistens für eine stereoskopische Abbildung eines abzubildenden Objekts verwendet werden. Verschiedene Vergrösserungen würden in diesem Fall zu unbrauchbaren Ergebnissen führen.

Entsprechend sind auch die Vergrösserungsfaktoren der beiden Galilei-Systeme in den Strahlenwegen 16.1, 16.2 typischerweise gleich. In dem dargestellten Beispiel sind die Vergrösserungsfaktoren aller vier Galilei-Systeme in den vier Strahlenwegen 15.1, 15.2, 16.1, 16.2 gleich. Dadurch kann erreicht werden, dass die Abbildung mit den beiden Strahlenwegen 15.1, 15.2, welche beispielsweise durch ein Okular eines entsprechend ausgerüsteten Mikroskops betrachtet werden kann, im Wesentlichen der zusätzlichen Abbildung mit den beiden Strahlenwegen 16.1, 16.2 entspricht, welche beispielsweise auf einem Bildschirm eines an das Mikroskop angeschlossenen Computers dargestellt wird.

Ein Vergrösserungswechsler umfasst typischerweise zwei Strahlenwege für eine stereoskopische Abbildung sowie zwei zusätzliche Strahlenwege für eine zusätzliche Abbildung bzw. eine Beleuchtung des abzubildenden Objekts. Die Linsentrommel 1 wird nun in der in Figur 1 dargestellten ersten Stellung derart in einen solchen Vergrösserungswechsler eingefügt, dass jeder der Strahlenwege 15.1, 15.2 sowie der Strahlenwege 16.1, 16.2 mit jeweils einem Abschnitt der vier Strahlenwege des Vergrösserungswechsler zusammenfällt.

Im Gegensatz zu den Öffnungen 10.1, 10.2, 10.3, 10.4 sind die Öffnungen 11.1, 11.2, 11.3, 11.4 in der Seitenfläche 5.1 sowie die (nicht sichtbaren) Öffnungen in der gegenüberliegenden Seitenfläche 5.2 leer, d. h. es sind keine Linsen darin eingesetzt. Wird nun die Linsentrommel 1 um die Drehachse 6 um 90 Grad in eine zweite Stellung gedreht, fallen nicht mehr die Strahlenwege 15.1, 15.2, 16.1, 16.2, sondern die Strahlenwege 15.3, 15.4 und 16.3, 16.4 mit den jeweiligen Strahlenwegen des Vergrösserungswechslers zusammen. D. h. die Strahlenwege 15.3, 15.4 und 16.3, 16.4 des Vergrösserungswechslers verlaufen nicht mehr durch die Öffnungen 10.1, 10.2, 10.3, 10.4 in den Seitenflächen 4.1, 4.2, sondern durch die Öffnungen 11.1, 11.2, 11.3, 11.4 in den Seitenflächen 5.1, 5.2. Da sich in diesen Öffnungen keine Linsen befinden, wird die Abbildung nicht vergrössert und auch nicht verkleinert - es resultiert quasi ein Vergrösserungsfaktor eins (wobei die Gesamtvergrösserung des Mikroskops typischerweise verschieden von eins ist, da das Mikroskop weitere optische Komponenten umfasst, welche die Vergrösserung beeinflussen).

Auf diese Weise lassen sich mit einer Linsentrommel 1, wie sie in Fig. 1 dargestellt ist, drei verschiedene Vergrösserungen realisieren. Angenommen, das zu betrachtende Objekt befindet sich in der Darstellung gemäss Fig. 1 links hinter der Linsentrommel 1, verlaufen die vom Objekt ausgehenden Lichtstrahlen in Richtung des Pfeils 18 auf die Linsentrommel 1 zu, treten durch die Öffnungen in der Seitenfläche 4.2 in diese ein, durchqueren die entsprechenden Galilei-Systeme und treten durch die Öffnungen 10.1, 10.2, 10.3, 10.4 wieder aus der Linsentrommel 1 aus, wobei durch die Galilei-Systeme eine erste Vergrösserung realisiert wird. Wie hoch der entsprechende Vergrösserungsfaktor ist, hängt sowohl von den verwendeten Linsen als auch von deren Abstand ab und kann im Prinzip frei gewählt werden. Dreht man die Linsentrommel 1 nun um 90 Grad im Uhrzeigersinn oder im Gegenuhrzeigersinn, verlaufen die Lichtstrahlen durch die Öffnungen 11.1, 11.2, 11.3, 11.4 in der Seitenfläche 5.1 sowie die Öffnungen in der Seitenfläche 5.2. Da sich in diesen Öffnungen keine Linsen befinden, wird die Abbildung nicht vergrössert oder verkleinert - es resultiert quasi ein zweiter Vergrösserungsfaktor von eins. Dreht man die Linsentrommel 1 um 90 Grad weiter, verlaufen die vom Objekt ausgehenden Lichtstrahlen wiederum durch die Öffnungen in den Seitenflächen 4.1 und 4.2, diesmal jedoch in der Gegenrichtung, d. h. sie treten durch die Öffnungen 10.1, 10.2, 10.3, 10.4 in der Seitenfläche 4.1 in die Linsentrommel 1 ein, durchqueren die entsprechenden Galilei-Systeme in umgekehrter Richtung und treten durch die Öffnungen in der Seitenfläche 4.2 wieder aus der Linsentrommel 1 aus. Auf diese Weise wird durch die Galilei-Systeme eine dritte Vergrösserung realisiert, welche wiederum von den verwendeten Linsen und deren Abstand abhängt.

Fig. 2 zeigt zur besseren Übersicht eine schematische Darstellung der Linsentrommel 1 aus Fig. 1. Gleiche Teile sind hierbei mit gleichen Bezugszeichen versehen.

Da in den Öffnungen 11.1, 11.2, 11.3, 11.4 der Seitenflächen 5.1, 5.2 ohnehin keine Linsen eingesetzt sind, wurden diese Seitenflächen 5.1, 5.2 in der schematischen Darstellung in Fig. 2 im Unterschied zur Darstellung in Fig. 1 weggelassen.

Fig. 3 wiederum zeigt eine schematische Darstellung einer weiteren Linsentrommel 21 für fünf Vergrösserungsstufen. Hier ist die Linsentrommel 21 aus der Sicht in Richtung der Drehachse 6, welche folglich als Punkt in der Mitte zu erkennen ist, d. h. in einer Art Schnittdarstellung gezeigt.

Die Grundform dieser Linsentrommel 21 ist nicht quadratisch wie bei der Linsentrommel 1 aus Fig. 1, sondern sechseckig. Die Linsentrommel 21 ist daher als regelmässiges Sechseckprisma mit drei Paaren von sich gegenüberliegenden Seitenflächen 24.1, 24.2, 25.1, 25.2, 26.1, 26.2 ausgebildet.

In den Seitenflächen 24.1, 24.2 befinden sich wiederum Öffnungen, in welche Linsen 32.1, 32.2, 32.3, 32.4 bzw. 33.1, 33.2, 33.3, 33.4 eingesetzt sind. Auch hier bilden je zwei Linsen, nämlich die Linsen 32.1 und 33.1, 32.22 und 33.2, 32.3 und 33.3 sowie 32.4 und 33.4 jeweils ein Galilei-System und definieren quasi die Strahlenwege 35.1, 35.2, 36.1 und 36.2.

Analog befinden sich in den Seitenflächen 26.1, 26.2 Öffnungen, in welche Linsen 42.1, 42.2, 42.3, 42.4 bzw. 43.1, 43.2, 43.3, 43.4 eingesetzt sind. Auch hier bilden die Linsen 42.1 und 43.1, 42.22 und 43.2, 42.3 und 43.3 sowie 42.4 und 43.4 jeweils ein Galilei-System und definieren so die Strahlenwege 45.1, 45.2, 46.1 und 46.2.

Um darzustellen, dass die Vergrösserung der Galilei-Systeme der Seitenflächen 24.1, 24.2 bzw. 26.1, 26.2 unterschiedlich sind, sind die Linsen 32.1, 32.2, 32.3, 32.4 bzw. 33.1, 33.2, 33.3, 33.4 dicker dargestellt als die Linsen 42.1, 42.2, 42.3, 42.4 bzw. 43.1, 43.2, 43.3, 43.4.

Schliesslich sind auch hier in die Öffnungen der Seitenflächen 25.1, 25.2 keine Linsen eingesetzt, sodass die Lichtstrahlen vom Objekt die Linsentrommel 21 entlang der Strahlenwege 55.1, 55.2, 56.1 und 56.2 ungehindert passieren können.

Durch jeweils zwei sich gegenüberliegende Seitenflächen 24.1, 24.2 bzw. 25.1, 25.2 bzw. 26.1, 2.6 verlaufen die entsprechenden Paare von Strahlenwegen 35.1, 35.2 bzw. 45.1, 45.2 bzw. 55.1, 55.2 sowie die jeweils zugehörigen zusätzlichen Strahlenwege 36.1, 36.2 bzw. 46.1, 46.2 bzw. 56.1, 56.2

Mit dieser Linsentrommel 21 können folglich fünf verschiedene Vergrösserungen realisiert werden. Nämlich zwei verschiedene Vergrösserungen mit den Galilei-Systemen in den Seitenflächen 24.1, 24.2, d. h. eine in jeder Richtung. Zwei weitere Vergrösserungen mit den Galilei-Systemen in den Seitenflächen 26.1, 26.2 sowie die Vergrösserung mit dem Faktor 1, wenn die Linsentrommel 21 so gedreht wird, dass sich die Strahlenwege 55.1, 55.2, 56.1 und 56.2 durch die Seitenflächen 25.1, 25.2 im Strahlengang vom Objekt zum Betrachter befinden.

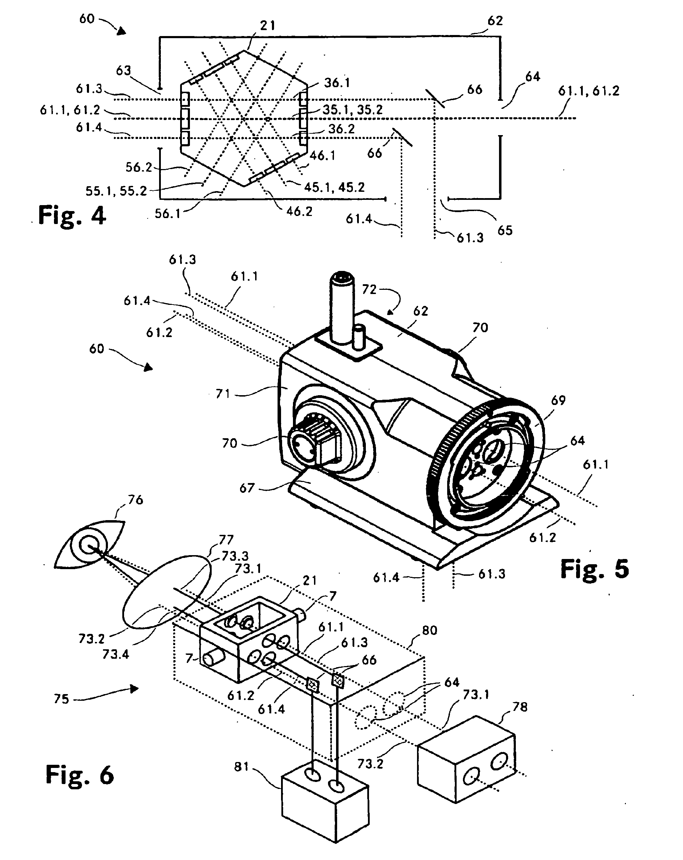

In Fig. 4 ist schematisch ein Vergrösserungswechsler 60 gemäss der Erfindung in einem Gehäuse 62 dargestellt, welcher eine Linsentrommel 21 gemäss Fig. 3 umfasst. Weiter sind vier Strahlenwege 61.1, 61.2, 61.3 und 61.4 dargestellt. Die Strahlenwege 61.1, 61.2 dienen für eine stereoskopische Abbildung und die Strahlenwege 61.3 und 61.4 sind die zusätzlichen, den beiden Strahlenwegen 61.1, 61.zugeordneten, Strahlenwege. Das Gehäuse 62 umfasst eine objektseitig angeordnete Öffnung, nachfolgend als Eintrittsöffnung 63 bezeichnet, sowie eine erste beobachterseitig angeordnete Öffnung, nachfolgend als Austrittsöffnung 64 bezeichnet und eine zweite beobachterseitig angeordnete Öffnung, nachfolgend als Austrittsöffnung 65 bezeichnet. (Obwohl Lichtstrahlen diese Öffnungen zur Beleuchtung oder Beobachtung in beiden Richtungen passieren können, werden diese Öffnungen als Eintritts- bzw. Austrittsöffnungen bezeichnet.) Die Eintrittsöffnung 63 hierbei eine einzelne grosse Öffnung, sodass sämtliche Strahlenwege 61.1, 61.2, 61.3 und 61.4 durch diese gemeinsame Öffnung in das Gehäuse 62 des Vergrösserungswechslers 60 eintreten können. In diese Eintrittsöffnung 63 ist im dargestellten Beispiel direkt eine Linse eingesetzt, welche als Frontlinse des zugehörigen optischen Untersuchungsgerätes dient. Die Eintrittsöffnung 63 kann aber auch mehrere kleine Öffnungen aufweisen, sodass jeweils eine separate Öffnung für jeden Strahlenweg 61.1, 61.2, 61.3, 61.4 vorhanden ist. Selbstverständlich können diese beiden Möglichkeiten auch gemischt werden, sodass mehrere Öffnungen für jeweils einen oder mehrere Strahlenwege vorhanden sind.

Entsprechendes gilt selbstverständlich auch für die Austrittsöffnungen 64, 65, wobei die Strahlenwege 61.1, 61.2 durch die erste Austrittsöffnung 64 und die Strahlenwege 61.3, 61.4 durch die zweite Austrittsöffnung 65 verlaufen.

Bei dem dargestellten Beispiel in Fig. 4 treten die Lichtstrahlen von dem abzubildenden Objekt alle parallel durch die Eintrittsöffnung 63, d. h. die Front- bzw. Objektivlinse, in den Vergrösserungswechsler 60 ein, durchqueren die Linsentrommel 21 und treten parallel wieder aus der Linsentrommel 21 aus. Die vom Objekt ausgehenden Lichtstrahlen, die die Linsentrommel 21 entlang der Strahlenwege 61.1, 61.2 durchqueren, treten durch die erste Austrittsöffnung 64 wieder parallel aus dem Vergrösserungswechsler 60 aus. Im Gegensatz dazu treten die vom Objekt ausgehenden Lichtstrahlen, die die Linsentrommel 21 entlang der Strahlenwege 61.3, 61.4 durchqueren, durch die zweite Austrittsöffnung 65 aus dem Vergrösserungswechsler 60 aus. Dieser umfasst hierfür zwei Spiegel 66, wobei je einer der Spiegel 66 in jedem der Strahlenwege 61.3, 61.4 positioniert ist. Da sich die Strahlenwege 61.3, 61.4 im Wesentlichen übereinander befinden, sind die Spiegel 66 in Richtung der Strahlenwege versetzt hintereinander angeordnet, damit die umgelenkten Lichtstrahlen nicht zusammenfallen. Die Spiegel 66 sind hierbei so angeordnet, dass die Lichtstrahlen rechtwinklig umgelenkt werden, d. h. die Spiegel sind in Bezug auf die Strahlenwege 61.3, 61.4 in einem Winkel von 45 Grad angeordnet. Es wäre jedoch auch möglich, die Spiegel 66 in einem anderen Winkel anzuordnen, oder andere Umlenkmittel wie beispielsweise Prismen zu verwenden, um die Strahlenwege 36.1, 36.2 zu einer beliebig positionierten zweiten Austrittsöffnung 65 umzulenken. Allerdings ist es auch in diesen Fällen von Vorteil, wenn die Strahlenwege 61.3, 61.4 auch nach der Umlenkung parallel verlaufen, sodass sie für eine zusätzliche, stereoskopische Abbildung des abzubildenden Objekts verwendet werden können.

Es wäre auch möglich, dass die Spiegel 66 dazu verwendet werden, um die Strahlenwege 61.1, 61.2 zur zweiten Austrittsöffnung 65 umzulenken, wobei die Strahlenwege 61.3, 61.4 weiter parallel verlaufen und durch die erste Austrittsöffnung 64 aus dem Gehäuse 62 des Vergrösserungswechslers 60 austreten würden.

Um Strahlübergänge der vom Objekt ausgehenden Lichtstrahlen zwischen den verschiedenen Strahlenwegen 61.1, 61.2, 61.3, 61.4 zu verhindern, können auch geeignete Blenden (nicht dargestellt) vorgesehen werden. Diese können an einem beliebigen Ort entlang dieser Strahlenwege 61.1, 61.2, 61.3, 61.4 angeordnet sein, d. h. sowohl ausserhalb wie innerhalb des Vergrösserungswechslers 60, wie auch ausserhalb oder innerhalb der Linsentrommel 21.

Fig. 5 zeigt wiederum den Vergrösserungswechsler 60 aus Fig. 4, hier in einer detaillierteren, perspektivischen Darstellung mit den ein- bzw. austretenden Strahlenwegen 61.1, 61.2, 61.3, 61.4 . Das Gehäuse 62 umfasst einen Sockel 67, mit welchem der Vergrösserungswechsler 60 beispielsweise auf einem Stativ befestigt werden kann, wobei auch dieser Sockel 67 eine Öffnung (nicht sichtbar) umfasst, durch welche die durch die zweite Austrittsöffnung 65 austretenden Lichtstrahlen entlang der Strahlenwege 61.3, 61.4 den Sockel 67 ungehindert passieren können.

Das Gehäuse 62 ist im Wesentlichen quaderförmig ausgebildet, wobei jenes Ende mit der Austrittsöffnung 64, welche hier als zwei separate Austrittsöffnungen 64 für die Strahlenwege 61.1, 61.2 erkennbar sind, einen Bajonett-Verschluss 69 umfassen. Mittels dieses Bajonett-Verschlusses 69 kann der Vergrösserungswechsler 60 mit der nachfolgenden optischen Einheit, etwa einem entsprechenden Beobachtungstubus wie z. B. einem Binokulartubus, zusammengeschaltet werden. Das Gehäuse 62 kann auch an jenem Ende mit der Eintrittsöffnung 63 (in Fig. 5 nicht sichtbar), einen derartigen Bajonett-Verschluss umfassen, um den Vergrösserungswechsler 60 mit einer vorangehenden optischen Einheit, beispielsweise einem Objektiv, zu verbinden. D. h. durch diese BajonettVerschlüsse kann der Vergrösserungswechsler wahlweise in ein optisches Untersuchungsgerät eingesetzt werden, wobei dies insbesondere in einem Abschnitt erfolgt, in welchem die Lichtstrahlen vom Objekt parallel zueinander verlaufen.

Das Gehäuse 62 umfasst an seiner vorderen Seitenfläche 71 einen Drehschalter 70, mit welchem sich die im Innern des Gehäuses 62 befindliche Linsentrommel 21 um ihre Drehachse 6 drehen lässt, um die gewünschte Vergrösserungsstufe einzustellen. Die Linsentrommel 21 ist hierfür beispielsweise mit ihren Achsstücken 7 in entsprechenden Öffnungen des Gehäuses 62 gelagert. Damit der Operateur die gewünschte Vergrösserung wahlweise mit der linken oder der rechten Hand einstellen kann, ist auch auf der der Seitenfläche 71 gegenüberliegenden Seitenfläche 72 ein entsprechender Drehschalter 70 vorgesehen.

Fig. 6 zeigt schematisch den Strahlengang in einem erfindungsgemässen Untersuchungsgerät, in diesem Fall in einem Stereomikroskop 75 zur Untersuchung eines Auges 76 mit zwei stereoskopischen Strahlenwegen 73.1 und 73.2. Der hier verwendete Vergrösserungswechsler 80 (gestrichelt dargestellt) umfasst eine Linsentrommel 21 wie in Fig. 2 dargestellt. Weiter ist die Objektivlinse 77 des Stereomikroskops 75 dargestellt, welche wie weiter oben beschrieben in die Eintrittsöffnung der Wechselvorrichtung eingesetzt ist. Diese Objektivlinse 77 verändert den Strahlenverlauf der von einem bestimmten Punkt des Auges 76 ausgehenden Lichtstrahlen derart, dass diese hinter der Objektivlinse 77 (vom Auge 76 aus gesehen) parallel verlaufen. Diese parallelen Lichtstrahlen durchqueren das Stereomikroskop 75 entlang der Strahlenwege 73.1, 73.2, 73.3, 73.4. Entsprechend durchqueren sie den Vergrösserungswechsler 80 entlang der Strahlenwege 61.1 und 61.2 bzw. 61.3 und 61.4, wobei die entsprechenden Abbildungen (oder Beleuchtungen) gemäss der aktuellen Stellung der Linsentrommel 21 erfolgen. Die Lichtstrahlen verlassen die Linsentrommel 21 wiederum parallel, wobei die entlang den Strahlenwegen 73.3, 73.4 bzw. 61.3, 61.4 verlaufenden Lichtstrahlen durch die beiden Spiegel 66 nach unten umgelenkt werden, sodass sie durch entsprechende Öffnungen auf der Unterseite des Vergrösserungswechslers 80 austreten (bzw. von unten durch die Öffnungen eintreten und von den beiden Spiegeln 66 in Richtung des Auges 76 umgelenkt werden). Die entlang den Strahlenwegen 73.1, 73.2 bzw. 61.1, 61.2 verlaufenden Lichtstrahlen treten aus den Austrittsöffnungen 64 aus und werden zum Binokulartubus 78 des Stereomikroskops 75 geleitet. Der Binokulartubus 78 umfasst pro Strahlenweg 73.1, 73.2 bzw. 61.1, 61.2 je eine (nicht dargestellte) Sammellinse, welche aus den parallelen Lichtstrahlen in der Brennebene dieser Sammellinse ein Bild erzeugt, welches durch die Okulare (nicht dargestellt) betrachtet werden kann.

Die entlang den Strahlenwegen 61.3, 61.4 verlaufenden, von den Spiegeln 66 umgelenkten Lichtstrahlen, werden ausserhalb des Vergrösserungswechslers 80 auf ein digitales Ophthalmoskop 81 geführt.

Wie der Vergrösserungswechsler 60 aus Fig. 5, umfasst auch der Vergrösserungswechsler 80 wenigstens einen Drehschalter 70, welcher mit den Achsstücken 7 derart zusammenwirkt, dass die Linsentrommel 21 durch Drehen des Drehschalters um die Drehachse 6 der Linsentrommel 21 gedreht werden kann. Indem die Linsentrommel 21 um ihre Drehachse 6 gedreht wird, kann in jeden der Strahlenwege 73.1, 73.2, 73.3, 73.4 bzw. 61.1, 61.2, 61.3, 61.4 jeweils ein Galilei-System eingefügt bzw. aus dem betreffenden Strahlenweg entfernt werden. D. h. die Vergrösserungen in sämtlichen Strahlenwegen 73.1, 73.2, 73.3, 73.4 bzw. 61.1, 61.2, 61.3, 61.4 werden durch die Drehung der Linsentrommel 21 zeitgleich umgeschaltet.

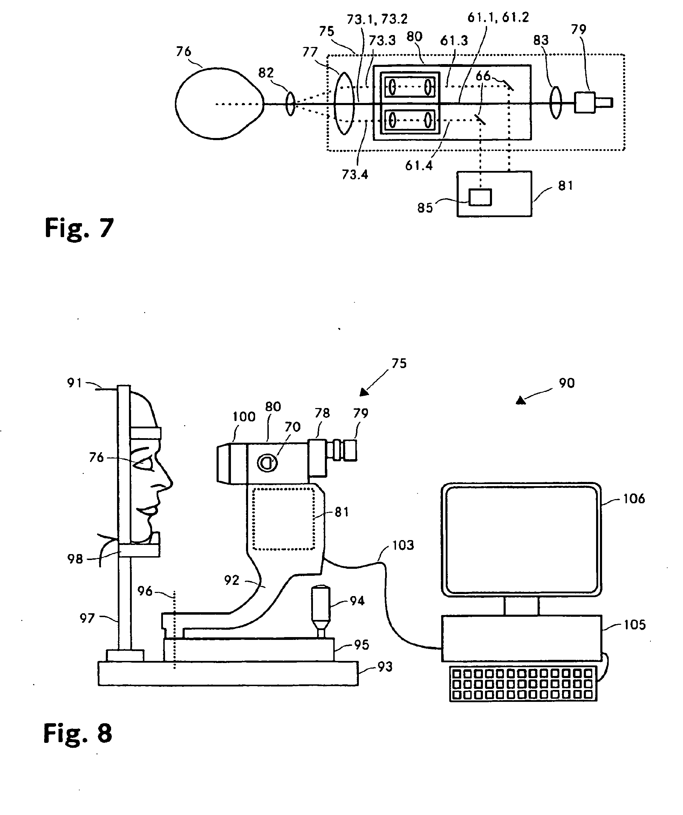

Fig. 7 zeigt wiederum das Stereomikroskop 75 zur Untersuchung des Auges 76 mit dem daran angeschlossenen digitalen Ophthalmoskop 81, diesmal in einer anderen schematischen Darstellung. In dieser Darstellung ist zusätzlich zur Objektivlinse 77 eine Vorsatzlinse 82 eingezeichnet, welche beispielsweise die Abbildung des Augenhintergrundes des Auges 76 ermöglicht. Weiter sind hier die Sammellinsen 83 zur Erzeugung der stereoskopischen Abbildung dargestellt, welche mittels der Okulare 79 betrachtet werden kann.

Das digitale Ophthalmoskop 81 verfügt über eine Lichtquelle 85, deren Licht via den Strahlenweg 61.4 bzw. 73.4 zum Auge 76 geführt und zur Beleuchtung des Auges 76 verwendet wird. Die vom Auge 76 reflektierten Lichtstrahlen gelangen (unter anderem) via die Strahlenwege 73.1, 73.2 bzw. 61.1, 61.2 und die Sammellinse 83 zu den Okularen 79. Die vom Auge 76 reflektierten Lichstrahlen gelangen aber auch via den Strahlenweg 73.3 bzw. 61.3 wieder zum digitalen Ophthalmoskop 81, welches daraus beispielsweise Live-Bilder des Auges 76 produziert und beispielsweise auf einem Computermonitor darstellt.

Dies ist beispielsweise in Fig. 8 zu erkennen, welche schematisch eine mögliche Realisierung eines Stereomikroskops 75 mit einem daran angeschlossenen digitalen Ophthalmoskop 81 zeigt. Fig. 8 zeigt eine Untersuchungsvorrichtung 90 zur Untersuchung des Auges 76 eines Patienten 91. Die Untersuchungsvorrichtung 90 umfasst ein Stereomikroskop 75, welches auf einem L-förmigen Haltearm 92 sitzt, der auf einem Tisch 93 befestigt ist. Der Haltearm 92 ist um die Achse 96 drehbar mit einem Kreuzschlitten 95 verbunden, welcher sich mit Hilfe des Hebels 94 in allen drei Raumrichtungen bewegen und präzise an einer gewünschten Position auf dem Tisch 93 positionieren lässt. Auf dem Tisch 93 ist weiter eine Halterung 97 mit einer Kinnstütze 98 befestigt, wobei der Kopf des Patienten 91 so in der Halterung 97 positioniert wird, dass das Kinn des Patienten 91 auf der Kinnstütze 98 abgestützt ist.

Mit dem Stereomikroskop 75 lässt sich nun das Auge 76 des Patienten 91, bzw. ein Abschnitt davon, betrachten und untersuchen. Das Stereomikroskop 75 umfasst ein Objektiv 100 mit der Objektivlinse 77 (nicht sichtbar), einen Vergrösserungswechsler 80 mit einem Drehschalter 70, wie er beispielsweise in den Fig. 4 und 5 dargestellt ist, einen Binokulartubus 78 sowie zwei Okulare 79.

Das digitale Ophthalmoskop 81 ist in dem dargestellten Beispiel direkt in den oberen Teil des Haltearms 92 integriert, sodass das Stereomikroskop 75 bei der Montage auf dem Haltearm 92 zugleich auch an das digitale Ophthalmoskop 81 angeschlossen wird.

Das digitale Ophthalmoskop 81 ist weiter über ein Kabel 103 mit einem Computer 105 verbunden, auf dessen Bildschirm 106 beispielsweise die vom digitalen Ophthalmoskop 81 aufgenommenen Bilder dargestellt werden können.

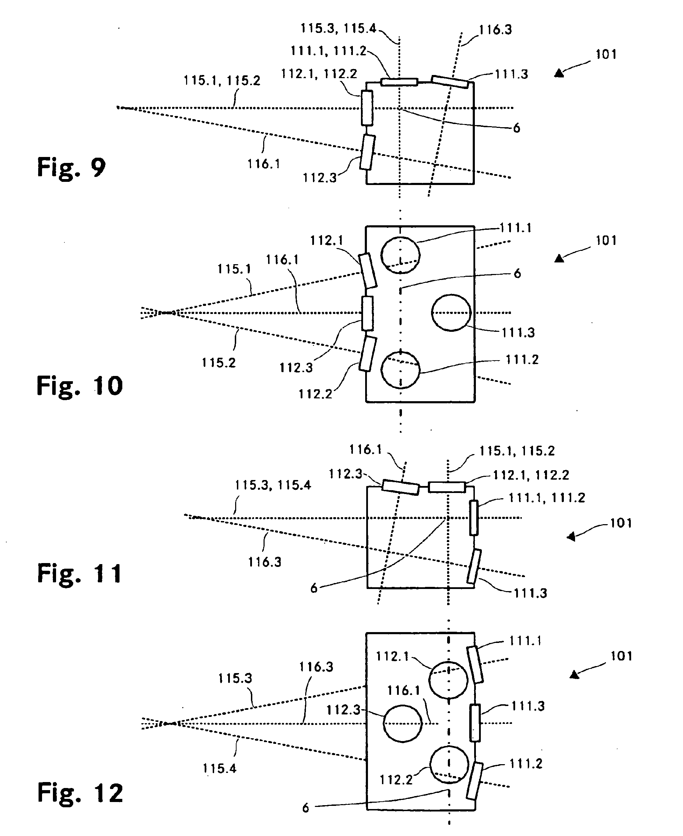

In den Figuren 9 bis 12 ist ein weiteres Beispiel für eine Linsentrommel 101 für eine erfindungsgemässe Wechsleroptik dargestellt. Auch in diesem Fall dient die Linsentrommel 101 zum Wechseln der Vergrösserung der Abbildung, allerdings nicht für ein Mikroskop nach dem Fernrohrprinzip, sondern für ein Greenough-Mikroskop. In den Darstellungen der Fig. 9 bis 12 befindet sich das abzubildende Objekt jeweils links von der Linsentrommel 101. Die Linsentrommel 101 umfasst in einer ihrer Seitenflächen drei in entsprechende Öffnungen eingesetzte Abbildungsoptiken 112.1, 112.2, 112.3, durch welche Strahlenwege 115.1, 115.2 und 116.1 verlaufen bzw. definiert werden. Weiter umfasst die Linsentrommel 101 in andern ihrer Seitenflächen drei weitere Abbildungsoptiken 111.1, 111.2, 111.3, durch welche Strahlenwege 115.3, 115.4 und 116.3 verlaufen bzw. definiert werden.

Die Strahlenwege 115.1, 115.2 bilden ein erstes Paar von Strahlenwegen für eine stereoskopische, erste Abbildung des Objekts mit einer bestimmten Vergrösserung und der Strahlenweg 116.1 ist ein zusätzlicher, den Strahlenwegen 115.1, 115.2 zugeordneter Strahlenweg für eine zusätzliche Abbildung bzw. eine Beleuchtung des Objekts mit entsprechenden Abbildungseigenschaften. Die Strahlenwege 115.3, 115.4 bilden ein zweites Paar von Strahlenwegen für eine stereoskopische, erste Abbildung des Objekts mit einer anderen Vergrösserung als die Abbildung entlang der Strahlenwege 115.1, 115.2 und der Strahlenweg 116.3 ist ein zusätzlicher, den Strahlenwegen 115.3, 115.4 zugeordneter Strahlenweg für eine zusätzliche Abbildung bzw. eine Beleuchtung des Objekts.

Fig. 9 zeigt die Linsentrommel 101 schematisch in einer Seitenansicht einer ersten Stellung, in welcher die Strahlenwege 115.1, 115.2, 116.1 aktiv, d. h. in den Strahlengang der zugehörigen Optikkomponente geschaltet sind. Fig. 10 zeigt die Linsentrommel 101 in dieser ersten Stellung in einer schematischen Ansicht von oben.

Fig. 11 zeigt die Linsentrommel 101 schematisch in einer Seitenansicht einer zweiten Stellung, in welcher die Strahlenwege 115.3, 115.4, 116.3 aktiv, d. h. in den Strahlengang der zugehörigen Optikkomponente geschaltet sind und Fig. 12 zeigt die Linsentrommel 101 in dieser zweiten Stellung in einer schematischen Ansicht von oben.

Dargestellt ist jeweils auch die Drehachse 6 (in den Fig. 9 und 11 als Punkt undin den Fig. 10 und 12 als Linie), um welche die Linsentrommel 101 von der ersten in die zweite Stellung und umgekehrt gedreht werden kann. Im dargestellten Beispiel wird die Linsentrommel 101 von der in den Fig. 9 und 10 gezeigten ersten Stellung um 90 Grad im Uhrzeigersinn in die in den Fig. 11 und 12 gezeigte, zweite Stellung gedreht.

Obwohl im Zusammenhang mit den Zeichnungen die Linsentrommel hauptsächlich als Vergrösserungswechsler beschrieben worden ist, kann die Wechsleroptik auch derart ausgebildet sein, dass damit nicht verschiedene Abbildungsvergrösserungen erreicht werden können, sondern dass je nach Stellung der Wechsleroptik andere Abbildungseigenschaften variiert werden können. So kann beispielsweise das Beleuchtungsspektrum für die Beleuchtung des abzubildenden Objekts variiert werden, indem etwa die in der Wechsleroptik verwendeten Abbildungsoptiken unterschiedliche Spektralbereiche durchlassen bzw. herausfiltern. Es kann aber auch die Beleuchtunsstärke oder die Grösse des ausgeleuchteten Bereichs des abzubildenden Objekts varriert werden. Die Beleuchtung kann beispielsweise eine flächenhafte Ausleuchtung des Auges umfassen, aber auch eine mit einem für die jeweilige Anwendung zweckmässigen Muster wie z. B. mit kleinen Kreisflächen, mit einem Streifenmuster oder auch mit einem einzelnen Spalt erfolgen, wobei ein solches Muster zeitlich und/oder örtlich unveränderlich oder auch variierbar sein kann. Natürlich ist es auch möglich, unterschiedliche Mess- oder Behandlungsstrahlen vorzusehen, beispielsweise mittels kohärenter Strahlung hoher Energiedichte (Laserstrahlung), z. B. für die Abtastung und/oder Behandlung bestimmter Bereiche im oder am Auge. Die Wechsleroptik kann aber auch zum Variieren anderer Abbildungseigenschaften ausgebildet sein. Je nach Stellung kann beispielsweise die Grösse des erzeugten Bildes oder auch die Helligkeit der Abbildung variiert werden. Es ist auch möglich die Wechsleroptik derart auszubilden, dass je nach deren Stellung unterschiedliche Wellenlängen absorbiert werden. Um diese unterschiedlichen Abbildungs- bzw. Beleuchtungseigenschaften zu erreichen, können die in die jeweiligen Strahlenwege der Wechsleroptik eingefügten Abbildungsoptiken (vorgängig hauptsächlich als Linsen bzw. Linsenanordnungen zum Ändern der Abbildungsvergrösserung bezeichnet), durch Abbildungsoptiken mit den gewünschten Eigenschaften ersetzt werden.

Natürlich ist es auch möglich, die Wechsleroptik derart auszubilden, dass zwei oder mehr dieser Eigenschaften in Kombination geändert werden können.

Zusammenfassend ist festzustellen, dass mit der Erfindung eine kompakte, kostengünstige und einfach herzustellende Optikkomponente geschaffen wird, mit welcher zwei Strahlenwege für eine stereoskopische Abbildung eines Objekts sowie ein oder mehrere zusätzliche Strahlenwege für eine zusätzliche mono- oder stereoskopische Abbildung/en zur Betrachtung oder Weiterverarbeitung bzw. für eine Beleuchtung des Objekts bereitgestellt werden können. Hierbei steht für jede dieser Abbildungen jeweils die volle Lichtstärke zur Verfügung, d. h. es sind keine Strahlteiler notwendig, die das Licht, welches entlang eines Strahlenwegs verläuft, in zwei einzelne Strahlenwege auftrennen. Die verschiedenen Strahlenwege werden von Beginn weg separat geführt, sodass qualitativ hochwertige und lichtstarke Abbildungen bzw. Beleuchtungen resultieren. Durch Umschalten der Optikkomponente, beispielsweise durch Drehen der zugehörigen Wechsleroptik von einer Stellung in eine andere Stellung, können zudem für jede dieser Abbildungen jeweils eine Mehrzahl von verschiedenen Abbildungs- bzw. Beleuchtungseigenschaften gewählt werden. Das Einstellen bzw. Auswählen einer der möglichen Abbildungen kann sowohl für jeden der Strahlenwege separat erfolgen, sie kann aber bevorzugt auch durch Betätigung eines gemeinsamen Einstellmechanismus für alle Strahlenwege gleichzeitig erfolgen.

## Patentansprüche

1. Optikkomponente (80) mit einem Paar von Strahlenwegen (61.1, 61.2) für eine stereoskopische Abbildung und einer Wechsleroptik (21), welche zwei Paare von Strahlenwegen (15.1, 15.2 bzw. 15.3, 15.4) mit jeweils einer Abbildungsoptik für eine stereoskopische Abbildung umfasst, wobei die Wechsleroptik (21) derart beweglich in der Optikkomponente (80) befestigt ist, dass wahlweise die beiden Strahlenwege (15.1, 15.2 bzw. 15.3, 15.4) eines der Paare von Strahlenwegen der Wechsleroptik (21) in einen Strahlabschnitt der beiden Strahlenwege (61.1, 61.2) der Optikkomponente eingeführt wird, wobei die Optikkomponente (80) wenigstens ein, den beiden Strahlenwegen (61.1, 61.2) zugeordnetes, zusätzliches Paar von Strahlenwegen (61.3, 61.4) mit jeweils einer Abbildungsoptik für eine weitere stereoskopische Abbildung umfasst,
**dadurch gekennzeichnet, dass**
die Wechsleroptik (21) pro Paar von Strahlenwegen (15.1, 15.2 bzw. 15.3, 15.4) wenigstens einen, dem jeweiligen Paar zugeordneten und räumlich getrennt von diesen angeordneten, zusätzlichen Strahlenweg (16.1, 16.2 bzw. 16.3, 16.4) umfasst,
und die Wechsleroptik (21) derart ausgebildet ist, dass beim wahlweisen Einfügen der beiden Strahlenwege (15.1, 15.2 bzw. 15.3, 15.4) mit jeweils einer Abbildungsoptik eines der Paare von Strahlenwegen der Wechsleroptik in die beiden Strahlenwege (61.1, 61.2) der Optikkomponente gleichzeitig der diesem Paar zugeordnete, zusätzliche Strahlenweg (16.1, 16.2 bzw. 16.3, 16.4) der Wechsleroptik in einen Strahlabschnitt des zusätzlichen Strahlenweges (61.3, 61.4) der Optikkomponente eingefügt wird.

2. Optikkomponente nach Anspruch 1, wobei diese genau zwei, den beiden Strahlenwegen (61.1, 61.2) zugeordnete, zusätzliche Strahlenwege (61.3, 61.4) umfasst und die Wechsleroptik (21) pro Paar von Strahlenwegen (15.1, 15.2 bzw. 15.3,15.4) ebenfalls genau zwei, dem jeweiligen Paar von Strahlenwegen (15.1, 15.2 bzw. 15.3,15.4) zugeordnete, zusätzliche Strahlenwege (16.1, 16.2 bzw. 16.3, 16.4) umfasst, wobei die beiden Strahlenwege (15.1, 15.2 bzw. 15.3, 15.4) jedes Paares von Strahlenwegen der Wechsleroptik (21) eine erste Ebene und die beiden diesem Paar zugeordneten, zusätzlichen Strahlenwege (16.1, 16.2 bzw. 16.3, 16.4) jeweils eine zweite Ebene definieren, wobei die beiden Ebenen jeweils senkrecht zueinander stehen und eine Schnittgerade der beiden Ebenen jeweils zwischen den beiden Strahlenwegen (15.1, 15.2 bzw. 15.3, 15.4) des betreffenden Paares liegt.

3. Optikkomponente nach einem der Ansprüche 1-2, wobei die Wechsleroptik (21) für wenigstens eines der Paare von Strahlenwegen (15.1, 15.2 bzw. 15.3, 15.4) und wenigstens eines der Paare von zusätzlichen Strahlenwegen (16.1, 16.2 bzw. 16.3, 16.4) eine Abbildungsoptik (12.1, 12.2, 12.3, 12.4), insbesondere eine Linsenanordnung, umfasst.

4. Optikkomponente nach Anspruch 3, wobei die Wechsleroptik (21) eine um eine Drehachse (6) drehbare Trägervorrichtung (2) umfasst, an welcher sämtliche Abbildungsoptiken befestigt sind und welche Einstellmittel (7) umfasst, mit welchen die Wechsleroptik um die Drehachse drehbar, insbesondere manuell drehbar ist.

5. Optikkomponente nach einem der Ansprüche 1-4, wobei sie ein Gehäuse (62) umfasst und das Gehäuse (62) eine objektseitig angeordnete Öffnung (63) für die Strahlenwege (61.1, 61.2) der Optikkomponente sowie die zusätzlichen Strahlenwege (61.3, 61.4) der Optikkomponente, wenigstens eine erste, beobachterseitig angeordnete Öffnung (64) für die Strahlenwege (61.1, 61.2) der Optikkomponente und wenigstens eine beobachterseitig und von der ersten Austrittsöffnung (64) räumlich getrennt angeordnete, zweite Öffnung (65) für die zusätzlichen Strahlenwege (61.3, 61.4) umfasst.

6. Optikkomponente nach Anspruch 5, wobei sie zur Umlenkung der zusätzlichen Strahlenwege (61.3, 61.4) zur zweiten Öffnung (65) eine Umlenkvorrichtung aufweist, welche insbesondere einen Spiegel (66) umfasst.

7. Optikkomponente nach einem der Ansprüche 1 bis 6, wobei die beiden Strahlenwege (61.1, 61.2) der Optikkomponente in dem Strahlabschnitt, in welchen die beiden Strahlenwege (15.1, 15.2 bzw. 15.3,15.4) einfügbar sind, parallel verlaufen und auch die beiden Strahlenwege (15.1, 15.2 bzw. 15.3, 15.4) eines Paares von Strahlenwegen der Wechsleroptik parallel zueinander sowie parallel zu dem diesem Paar zugeordneten, zusätzlichen Strahlenweg (16.1, 16.2, bzw. 16.3, 16.4) verlaufen.

8. Optikkomponente nach Anspruch 7, wobei die Wechsleroptik (21) für wenigstens eines der Paare von Strahlenwegen (15.1, 15.2 bzw. 15.3, 15.4) eine für eine optische Abbildung von unendlich nach unendlich und für eine aufrechte Abbildung ausgebildete Linsenanordnung (12.1, 12.2, 12.3, 12.4) mit einer Sammellinse und einer Zerstreuungslinse umfasst.

9. Optikkomponente nach einem der Ansprüche 1 bis 8 für ein optisches Untersuchungsgerät, insbesondere ein Stereomikroskop (75), mit zwei Strahlenwegen für eine stereoskopische Abbildung eines abzubildenden Objekts (76), wobei die Optikkomponente wahlweise derart mit dem Untersuchungsgerät verbindbar ist, dass die Strahlenwege (61.1, 61.2) der Optikkomponente mit einem Abschnitt der Strahlenwege des Untersuchungsgeräts zusammenfallen.

10. Optisches Untersuchungsgerät, insbesondere ein Stereomikroskop (75), mit zwei Strahlenwegen für eine stereoskopische Betrachtung eines abzubildenden Objekts (76), **dadurch gekennzeichnet, dass** das Untersuchungsgerät (75) eine Optikkomponente (80) nach einem der Ansprüche 1 bis 9 umfasst, wobei die Optikkomponente derart mit dem Untersuchungsgerät verbunden ist, dass die Strahlenwege (61.1, 61.2) der Optikkomponente einen Abschnitt der Strahlenwege des Untersuchungsgeräts bilden.

11. Optisches Untersuchungsgerät nach Anspruch 10, wobei es eine Objektivlinse (77) und zwei Okulare (79) umfasst und die Optikkomponente (80) zwischen der Objektivlinse (77) und den Okularen (79) in die Strahlenwege des optischen Untersuchungsgeräts eingefügt ist.

12. Optisches Untersuchungsgerät nach einem der Ansprüche 10 bis 11, wobei dieses eine optische Abbildungsvorrichtung (81) umfasst, und der wenigstens eine zusätzliche Strahlenweg (36.1, 36.2) der Optikkomponente (80) mit einer Umlenkvorrichtung, welche insbesondere einen Spiegel (66) umfasst, zur Abbildungsvorrichtung (81) umlenkbar ist.

13. Optisches Untersuchungsgerät nach Anspruch 12, wobei es Beleuchtungsmittel (85) zur Beleuchtung des abzubildenden Objekts (76) mittels des wenigstens einen zusätzlichen Strahlenwegs (61.3, 61.4) umfasst.

## Claims

1. Optical component (80) having a pair of beam paths (61.1, 61.2) and a changing optics (21) which comprises two pairs of beam paths (15.1, 15.2 and 15.3, 15.4, respectively), each having an imaging optic for a stereoscopic image, the changing optics (21) being movably fastened in the optical component (80) in such a way that the two beam paths (15.1, 15.2 and 15.3, 15.4, respectively) of one of the pairs of beam paths of the changing optics (21) are optionally inserted in a beam section of the two beam paths (61.1, 61.2) of the optical component, wherein the optical component (80) comprises at least one further pair of beam paths (61.3, 61.4) assigned to the two beam paths (61.1, 61.2) each having an imaging optic for a further stereoscopic image,
**characterized in that**
the changing optics (21) comprises per pair of beam paths (15.1, 15.2 and 15.3, 15.4, respectively) at least one additional beam path (16.1, 16.2 and 16.3, 16.4, respectively) assigned to the respective pair and spatially separated therefrom,
and the changing optics (21) is designed in such a way that, during the optional insertion of the two beam paths (15.1, 15.2 and 15.3, 15.4, respectively), each having an imaging optic, of one of the pairs of beam paths of the changing optics in the two beam paths (61.1, 61.2) of the optical component, the additional beam path (16.1, 16.2 and 16.3, 16.4, respectively) of the changing optics which is assigned to this pair is simultaneously inserted into a beam section of the additional beam path (61.3, 61.4) of the optical component.

2. Optical component according to Claim 1, in which the latter comprises exactly two additional beam paths (61.3, 61.4) assigned to the two beam paths (61.1, 61.2), and the changing optics (21) likewise comprises per pair of beam paths (15.1, 15.2 and 15.3, 15.4, respectively) exactly two additional beam paths (16.1, 16.2 and 16.3, 16.4, respectively) assigned to the respective pair of beam paths (15.1, 15.2 and 15.3, 15.4, respectively), the two beam paths (15.1, 15.2 and 15.3, 15.4, respectively) of each pair of beam paths of the changing optics (21) defining a first plane, and the two additional beam paths (16.1, 16.2 and 16.3, 16.4, respectively) assigned to this pair respectively defining a second plane, the two planes respectively being mutually perpendicular, and a line of intersection of the two planes respectively lying between the two beam paths (15.1, 15.2 and 15.3, 15.4, respectively) of the relevant pair.

3. Optical component according to one of Claims 1-2, in which the changing optics (21) comprises an imaging optics (12.1, 12.2, 12.3, 12.4), in particular a lens arrangement, for at least one of the pairs of beam paths (15.1, 15.2 and 15.3, 15.4, respectively) and at least one of the pairs of additional beam paths (16.1, 16.2 and 16.3, 16.4, respectively).

4. Optical component according to Claim 3, in which the changing optics (21) comprises a carrier device (2) which can be rotated about a rotation axis (6) and on which all the imaging optics are fastened and which comprises setting means (7) with the aid of which the changing optics can be rotated about the rotation axis, in particular can be rotated manually.

5. Optical component according to one of Claims 1-4, in which it comprises a housing (62), and the housing (62) comprises an aperture (63), arranged on the object side, for the beam paths (61.1, 61.2) of the optical component and for the additional beam paths (61.3, 61.4) of the optical component, at least one first aperture (64), arranged on the observer side, for the beam paths (61.1, 61.2) of the optical component, and at least one second aperture (65), arranged on the observer side and in a fashion spatially separated from the first exit aperture (64), for the additional beam paths (61.3, 61.4).

6. Optical component according to Claim 5, in which it has a deflecting device, which particularly comprises a mirror (66), for deflecting the additional beam paths (61.3, 61.4) to the second aperture (65).

7. Optical component according to one of Claims 1 to 6, in which the two beam paths (61.1, 61.2) of the optical component run parallel in the beam section into which the two beam paths (15.1, 15.2 and 15.3, 15.4, respectively) can be inserted, and the two beam paths (15.1, 15.2 and 15.3, 15.4, respectively) of one pair of beam paths of the changing optics also run parallel to one another and parallel to the additional beam path (16.1, 16.2 and 16.3, 16.4, respectively) assigned to this pair.

8. Optical component according to Claim 7, in which the changing optics (21) for at least one of the pairs of beam paths (15.1, 15.2 and 15.3, 15.4, respectively) comprises a lens arrangement (12.1, 12.2, 12.3, 12.4) designed for optically imaging from infinity to infinity and for direct imaging and having a positive lens and a diverging lens.

9. Optical component according to one of Claims 1 to 8 for an optical examination device, in particular a stereomicroscope (75), having two beam paths for stereoscopic imaging of an object (76) to be imaged, it being possible to connect the optical component optionally to the examination device in such a way that the beam paths (61.1, 61.2) of the optical component coincide with a section of the beam paths of the examination device.

10. Optical examination device, in particular a stereomicroscope (75), having two beam paths for stereoscopically observing an object (76) to be imaged, **characterized in that** the examination device (75) comprises an optical component (80) according to one of Claims 1 to 9, the optical component being connected to the examination device in such a way that the beam paths (61.1, 61.2) of the optical component form a section of the beam paths of the examination device.

11. Optical examination device according to Claim 10, in which it comprises an objective lens (77) and two eyepieces (79), and the optical component (80) is inserted into the beam paths of the optical examination device between the objective lens (77) and the eyepieces (79).

12. Optical examination device according to one of Claims 10 to 11, in which it comprises an optical imaging device (81), and the at least one additional beam path (36.1, 36.2) of the optical component (80) can be deflected to the imaging device (81) with the aid of a deflecting device that, in particular, comprises a mirror (66).

13. Optical examination device according to Claim 12 which comprises illuminating means (85) for illuminating the object (76) to be imaged by means of the at least one additional beam path (61.3, 61.4).

## Revendications

1. Composant optique (80) présentant
deux parcours de rayons (61.1, 61.2) servant à former une image stéréoscopique et
une optique remplaçable (21) qui comporte deux paires de parcours de rayons (15.1, 15.2 et 15.3, 15.4), chaque parcours de rayons dotées d'une optique de formation d'image en vue de former une image stéréoscopique,
l'optique remplaçable (21) étant fixée à déplacement dans le composant optique (80) de telle sorte que les deux parcours de rayons (15.1, 15.2 et 15.3, 15.4) de l'une des paires de parcours de rayons de l'optique remplaçable (21) puissent être insérés sélectivement dans une partie du rayon des deux parcours de rayons (61.1, 61.2) du composant optique,
le composant optique (80) comportant au moins une paire supplémentaire de parcours de rayons (61.3, 61.4) associée aux deux parcours de rayons (61.1, 61.2) et présentant différentes optiques de formation d'image en vue de former une autre image stéréoscopique, **caractérisé en ce que**
pour chaque paire de parcours de rayons (15.1, 15.2 et 15.3, 15.4), l'optique remplaçable (21) comporte au moins une paire supplémentaire de parcours de rayons (16.1, 16.2 et 16.3, 16.4) associée à chaque paire et séparés spatialement de ces dernières, et
**en ce que** l'optique remplaçable (21) est configurée de telle sorte que lors d'une insertion sélective des deux parcours de rayons (15.1, 15.2 et 15.3, 15.4), chaque parcours de rayons dotées d'une optique de formation d'image, l'une des paires de parcours de rayons de l'optique remplaçable soit insérée simultanément dans les deux parcours de rayons (61.1, 61.2) des composants optiques de la paire supplémentaire de parcours de rayons (16.1, 16.2 et 16.3, 16.4), associée à cette paire, de l'optique remplaçable dans une partie du rayon de la paire supplémentaire de parcours de rayons (61.3, 61.4) du composant optique.

2. Composant optique selon la revendication 1, comprenant exactement deux parcours de rayons supplémentaires (61.3, 61.4) associés aux deux parcours de rayons (61.1, 61.2), l'optique remplaçable (21) présentant également pour chaque paire de parcours de rayons (15.1, 15.2 et 15.3, 15.4) exactement deux parcours de rayons supplémentaires (16.1, 16.2 et 16.3, 16.4) associés à chaque paire de parcours de rayons (15.1, 15.2 et 15.3, 15.4), les deux parcours de rayons (15.1, 15.2 et 15.3, 15.4) de chaque paire de parcours de rayons de l'optique remplaçable (21) définissant un premier plan et les deux parcours supplémentaires de rayons (16.1, 16.2 et 16.3, 16.4) associés à cette paire définissant chacun un deuxième plan, les deux plans étant perpendiculaires l'un à l'autre et la droite d'intersection des deux plans étant située entre les deux parcours de rayons (15.1, 15.2 et 15.3, 15.4) de la paire concernée.

3. Composant optique selon l'une des revendications 1 et 2, dans lequel l'optique remplaçable (21) comporte une optique (12.1, 12.2, 12.3, 12.4) de formation d'image, en particulier un ensemble de lentilles pour au moins l'une des paires de parcours de rayons (15.1, 15.2 et 15.3, 15.4) et au moins l'une des paires de parcours supplémentaires de rayons (16.1, 16.2 et 16.3, 16.4).

4. Composant optique selon la revendication 3, dans lequel l'optique remplaçable (21) comporte un dispositif de support (2) qui peut tourner autour d'un axe de rotation (6), sur lequel toutes les optiques de formation d'image sont fixées et qui comprend des moyens de réglage (7) qui permettent de faire tourner l'optique remplaçable autour de l'axe de rotation, en particulier manuellement.

5. Composant optique selon l'une des revendications 1 à 4, qui comporte un boîtier (62), le boîtier (62) présentant une ouverture (63), disposée du côté de l'objet, pour les parcours de rayons (61.1, 61.2) du composant optique ainsi que pour les parcours supplémentaires de rayons (61.3, 61.4) du composant optique, au moins une première ouverture (64), disposée du côté de l'observateur, pour les parcours de rayons (61.1, 61.2) du composant optique et au moins une deuxième ouverture (65), disposée du côté de l'observateur et séparée spatialement de la première ouverture de sortie (64), pour les parcours supplémentaires de rayons (61.3, 61.4).

6. Composant optique selon la revendication 5, présentant un dispositif de déviation qui comporte en particulier un miroir (66) pour dévier les parcours supplémentaires de rayons (61.3, 61.4) en direction de la deuxième ouverture (65).

7. Composant optique selon l'une des revendications 1 à 6, dans lequel les deux parcours de rayons (61.1, 61.2) du composant optique s'étendent parallèlement dans la partie du rayon dans laquelle les deux parcours de rayons (15.1, 15.2 et 15.3, 15.4) peuvent être insérés, les deux parcours de rayons (15.1, 15.2 et 15.3, 15.4) d'une paire de parcours de rayons de l'optique remplaçable s'étendant parallèlement l'un à l'autre ainsi que parallèlement au parcours supplémentaire de rayons (16.1, 16.2 et 16.3, 16.4) associé à cette paire.

8. Composant optique selon la revendication 7, dans lequel pour au moins l'une des paires de parcours de rayons (15.1, 15.2 et 15.3, 15.4), l'optique remplaçable (21) présente un ensemble de lentilles (12.1, 12.2, 12.3, 12.4) doté d'une lentille collectrice et d'une lentille divergente et configuré pour former des images optiques d'infini à infini et pour former d'une image droit.

9. Composant optique selon l'une des revendications 1 à 8, pour appareil d'examen optique, en particulier stéréomicroscope (75), et présentant deux parcours de rayons permettant de former une image stéréoscopique d'un objet (76) dont l'image doit être formée, le composant optique pouvant être relié sélectivement à l'appareil d'examen de telle sorte que les parcours de rayons (61.1, 61.2) du composant optique coïncident avec une partie des parcours de rayons de l'appareil d'examen.

10. Appareil d'examen optique, en particulier stéréomicroscope (75), présentant deux parcours de rayons permettant un examen stéréoscopique d'un objet (76) dont l'image doit être formée, **caractérisé en ce que** l'appareil d'examen (75) comporte un composant optique (80) selon l'une des revendications 1 à 9, le composant optique étant relié à l'appareil d'examen de telle sorte que les parcours de rayons (61.1, 61.2) du composant optique forment une partie du parcours des rayons de l'appareil d'examen.

11. Appareil d'examen optique selon la revendication 10, comprenant une lentille d'objectif (77) et deux oculaires (79), le composant optique (80) étant inséré entre la lentille d'objectif (77) et l'oculaire (79) dans le parcours des rayons de l'appareil d'examen optique.

12. Appareil d'examen optique selon l'une des revendications 10 à 11, comportant un dispositif optique (81) de formation d'image, le ou les parcours supplémentaires de rayons (36.1, 36.2) du composant optique (80) pouvant être déviés vers le dispositif (81) de formation d'image par un dispositif de déviation qui comporte en particulier un miroir (66).

13. Appareil d'examen optique selon la revendication 12, qui comporte des moyens d'éclairage (85) qui éclairent l'objet (76) dont l'image doit être formée au moyen du ou des parcours supplémentaires de rayons (61.3, 61.4).
